(19) Europäisches Patentamt
European Patent Office
Office européen des brevets

(11) Veröffentlichungsnummer: **0 069 289**
**B1**

# (12) EUROPÄISCHE PATENTSCHRIFT

(45) Veröffentlichungstag der Patentschrift: **10.10.84**

(51) Int. Cl.³: **C 07 D 249/08**, C 07 D 233/60, A 01 N 43/64, A 01 N 43/50

(21) Anmeldenummer: **82105596.9**

(22) Anmeldetag: **25.06.82**

## (54) Azolderivate, Verfahren zu ihrer Herstellung und diese enthaltende Fungizide.

(30) Priorität: **04.07.81 DE 3126478**

(43) Veröffentlichungstag der Anmeldung: **12.01.83 Patentblatt 83/2**

(45) Bekanntmachung des Hinweises auf die Patenterteilung: **10.10.84 Patentblatt 84/41**

(84) Benannte Vertragsstaaten: **AT BE CH DE FR GB IT LI NL SE**

(56) Entgegenhaltungen:
**EP-A-0 001 654**
**GB-A-2 011 396**
**US-A-4 210 656**
**US-A-4 210 657**

(73) Patentinhaber: **BASF Aktiengesellschaft, Carl-Bosch-Strasse 38, D-6700 Ludwigshafen (DE)**

(72) Erfinder: **Ruland, Alfred, Dr., Schriesheimer Strasse 11, D-6945 Hirschberg (DE)**
Erfinder: **Reuther, Wolfgang, Dr., Am Pferchelhang 16, D-6900 Heidelberg (DE)**
Erfinder: **Pommer, Ernst-Heinrich, Dr., Berliner Platz 7, D-6703 Limburgerhof (DE)**

EP 0 069 289 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue Azolderivate, Verfahren zu ihrer Herstellung und ihre Verwendung als Fungizide.

Es ist bekannt, daß Imidazolylether, z. B. das 1-(2-(2,4-Dichlorphenyl)-2-(2-propenyloxy)-ethyl-1H-imidazol (GB-PS 1 318 590) fungizide Wirksamkeit besitzen. Die Wirkung dieser Stoffe ist jedoch unbefriedigend.

Es ist ferner bekannt, daß
1-beta-(Methoxy-methoxy-2,4-dichlorphenäthyl)-imidazol-nitrat,
alpha-(2,4-Dichlorphenyl)-beta-imidazol-1-yl-vinyl-methoxymethylether und
1-(2,4-Dichlorphenyl)-1-(methoxmethoxy)-2-(imidazol-1-yl)-propen
fungizid wirksam sind (EP-A-1 654, GB-A-2 011 396, US-A-210 657).

Es wurde gefunden, daß Verbindungen der Formel 1

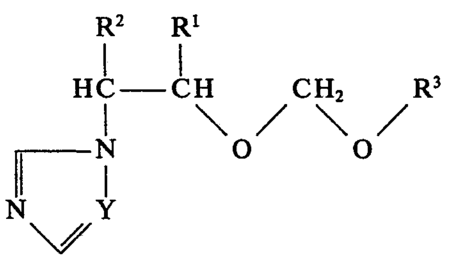

(1)

in der

$R^1$ tertiär Butyl oder Phenyl,
$R^2$ Wasserstoff oder Phenyl, Phenoxy oder Thiophenoxyl bedeutet, wobei der Phenyl-, Phenoxyl- oder Thiophenoxyl-Rest gegebenenfalls einfach oder mehrfach durch Halogen, Phenyl oder Phenoxy substituiert ist,
$R^3$ Methyl und
Y N bedeutet,

eine gute fungizide Wirkung haben und in unpolaren Lösungsmitteln sehr gut löslich sind.

$R^2$ bedeutet beispielsweise 4-Chlorphenoxy oder 4-Bromphenoxy.

Die neuen Verbindungen lassen sich beispielsweise aus Alkoholen, die nach bekannten Verfahren hergestellt werden können und in der Literatur beschrieben sind, darstellen. Man setzt zunächst den Alkohol mit Alkalihydriden, Alkaliamiden, metallorganischen Verbindungen wie Lithiumdiisopropylamid, Butyllithium bzw. Phenyllithium in aprotischen organischen Solventien wie Acetonitril, Dimethylformamid, Dimethylether, Tetrahydrofuran (THF), oder mit Basen wie Alkalihydroxiden im 2-Phasensystem (wäßrig-organisch) in Gegenwart von quartären Ammonium- bzw. Phosphoniumsalzen zu den Alkoholaten der Formel 2 um, worin $R^1$, $R^2$ und M die oben angegebene Bedeutung haben.

Diese reagieren mit Halogenethern 3, wobei $R^3$ die oben angegebene Bedeutung hat, vorzugsweise Chlorethern, in einem der genannten Lösungsmittel zu den Verbindungen der Formel 1.

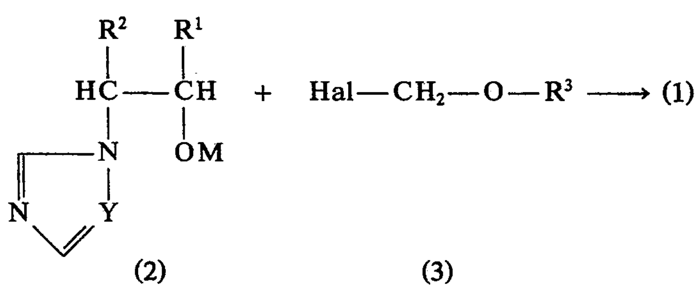

(2) (3)

Die Verbindungen der Formel 1 besitzen 2 (evtl. auch mehr) Chiralitätszentren. Sie fallen im Rahmen der Synthese als Diastereoisomerengemische an, sofern die Ausgangsalkohole ebenfalls als Diastereoisomerengemische eingesetzt werden. Durch fraktionierte Kristallisation oder durch Chromatographie an Kieselgel mit Methanol/Wasser lassen sich reine Diastereoisomere erhalten, die ebenso wie die Gemische von der Erfindung umfaßt werden.

## Beispiel 1

### 1-(1,2,4-Triazolyl-(1))-1-(4-chlorphenoxy)-2-methoxymethoxy-3,3-dimethylbutan (Verbindung Nr. 1)

Eine Lösung von 29,6 g (0,1 Mol) 1-(1,2,4-Triazolyl-(1))-1-(4-chlorphenoxy)-3,3 dimethylbutan-2-ol in 300 ml absolutem Tetrahydrofuran wurde unter Stickstoff 3 bis 4 h mit 2,4 g (0,1 Mol) Natriumhydrid umgesetzt. Danach gab man 12,1 g (0,15 Mol) Monochlordimethylether zu, rührt anschließend noch 1 Stunde, hydrolysierte mit 500 ml Wasser, extrahierte zweimal mit 500 ml Diethylether, trocknete über Natriumsulfat und destillierte das Lösungsmittel im Vakuum ab. Das so erhaltene Rohprodukt wurde zur weiteren Reinigung an Kieselgel mit Methanol/Wasser 3 : 1 (Gew.-Teile) chromatographiert. Man erhielt die gewünschte Verbindung als farbloses Öl in einer Ausbeute von 20,3 g (60% d. Th.).

## Beispiel 2

### 2-(1,2,4-Triazolyl-(1))-1-methoxymethoxy-1-phenylethan (Verbindung Nr. 7)

Zu einer Suspension von 2,4 g (0,1 Mol) Natriumhydrid in absolutem Tetrahydrofuran wurden 18,9 g (0,1 Mol) 2-(1,2,4-Triazolyl-(1))-1-phenylethan-1-ol unter Stickstoff zugegeben und 3 Stunden bei 30 bis 40°C gerührt. Anschließend gab man 12,6 g (0,15 Mol) Monochlordimethylether zu, rührte noch 1 Stunde, hydrolysierte mit 500 ml Wasser, extrahierte zweimal mit 500 ml Diethylether, trocknete über Natriumsulfat und destillierte das Lösungsmittel im Vakuum ab. Das Rohprodukt wurde zur weiteren Reinigung an Kieselgel mit Methanol/Wasser 3 : 1 chromatographiert. Die gewünschte Verbindung fiel als farbloses Öl in einer Ausbeute von 16,3 g (70% d. Th.) an.

In entsprechender Weise wurden die folgenden Verbindungen hergestellt.

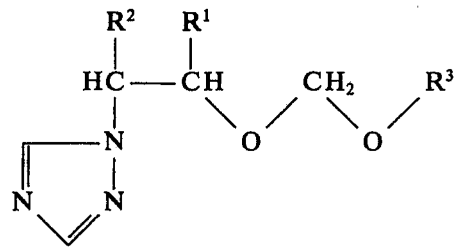

(1)

| Nr. | $R^1$ | $R^2$ | $R^3$ | Phys. Daten | $^1H$-NMR ($\delta$ in ppm; in $CDCl_3$) jeweils nur von einem Diastereoisomeren angegeben |
|---|---|---|---|---|---|
| 1 | tert.-Butyl | 4-Chlorphenoxy | Methyl | Öl | $\delta$ = 1,1 (s, 9H); 3,3 (s, 3H); 3,65 (d, 1H, J = 3 Hz); 4,1 (d, 1H, J = 6 Hz); 4,4 (d, 1H, J = 6 Hz); 6,35 (d, 1H, J = 3 Hz); 6,8–7,3 (AA'BB', 4 H); 7,9 (s, 1H); 8,25 (s, 1H) |
| 2 | tert.-Butyl | 4-tert.-Butylphenoxy | Methyl | Öl | $\delta$ = 1,15 (s, 9H); 1,3 (s, 9H); 3,3 (s, 3H); 3,65 (d, 1H, J = 3 Hz); 4,1 (d, 1H, J = 6 Hz); 4,4 (d, 1H, J=6 Hz); 6,4 (d, 1H, J=3 Hz); 6,8–7,35 (m, 4 H); 7,95 (s, 1H); 8,3 (s, 1H) |
| 3 | tert.-Butyl | 4-Bromphenoxy | Methyl | Öl | $\delta$ = 1 (s, 9H); 3,3 (s, 3H); 3,65 (d, 1H, J = 3 Hz); 4,1 (d, 1H, J = 6 Hz); 4,45 (d, 1H, J = 6 Hz); 6,3 (d, 1H, J = 3 Hz); 6,7–7,5 (AA'BB', 4 H); 8,05 (s, 1H); 8,35 (s, 1H) |
| 4 | tert.-Butyl | 2-Chlorphenoxy | Methyl | Öl | $\delta$ = 1,05 (s, 9H); 3,3 (s, 3H); 3,75 (d, 1H, J = 3 Hz); 4,4 (d, 1H, J = 6 Hz); 4,6 (d, 1H, J = 6 Hz); 6,4 (d, 1H, J = 3 Hz); 6,65–7,5 (m, 4 H); 7,9 (s, 1H); 8,3 (s, 1H) |
| 5 | tert.-Butyl | 4-Phenylphenoxy | Methyl | Öl | $\delta$ = 1,1 (s, 9H); 3,25 (s, 3H); 3,65 (d, 1H, J = 3 Hz); 4,1 (d, 1H, J = 6 Hz); 4,45 (d, 1H, J = 6 Hz); 6,4 (d, 1H, J = 3 Hz); 6,8–7,6 (m, 9 H); 7,9 (s, 1H); 8,25 (s, 1H) |
| 6 | tert.-Butyl | 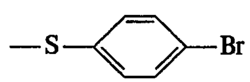 | Methyl | Öl | $\delta$ = 0,8 (s, 9H); 3,55 (s, 3H); 3,75 (d, 1H, J = 1 Hz); 4,9 (s, 2H); 5,85 (d, 1H, J = 1 Hz); 6,9–7,4 (AA'BB', 4 H); 7,7 (s, 1H); 8,6 (s, 1H) |
| 7 | Phenyl | H | Methyl | Öl | $\delta$ = 3 (s, 3H); 4,4 (d, 2H, J = 6 Hz); 4,5 (d, 2H, J = 1 Hz); 5,05 (t, 1H, J = 6 Hz); 7,3 (s, 5H); 7,9 (s, 1H); 8,1 (s, 1 H) |
| 8 | Phenyl | 4-Bromphenoxy | Methyl | Öl | $\delta$ = 3,15 (s, 3H); 4,5 (s, 2H); 5,3 (d, 1H, J = 6 Hz); 6,15 (d, 1H, J = 6 Hz); 6,5–7,5 (m, 9 H); 7,9 (s, 1H); 8,2 (s, 1H) |
| 9 | Phenyl | 4-Phenylphenoxy | Methyl | Öl | $\delta$ = 3,2 (s, 3H); 4,5 (s, 2H); 5,3 (d, 1H, J = 6 Hz); 6,2 (d, 1H, J = 6 Hz); 6,7–7,6 (m, 14H); 8 (s, 1H); 8,3 (s, 1H) |

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie in Gemüsen wie Gurken, Bohnen oder Kürbisgewächsen.

Die neuen Verbindungen sind insbesondere geeignet zur Bekämpfung folgender Pflanzenkrankheiten:

Erysiphe graminis (echter Mehltau) in Getreide,
Erysiphe cichoriacearum (echter Mehltau) an Kürbisgewächsen
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie
Helminthosporiumarten an Getreide,
Ustilago-Arten an Getreide und Zuckerrohr,
Rhynchosporium secale an Getreide und ganz besonders Venturia inaequalis (Apfelschorf).

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemäßen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Ketone (z. B. Cyclohexanon), Amine (z. B. Ethanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 95 Gew.-% Wirkstoff.

Die Aufwandmengen liegen je nach Art des gewünschten Effektes zwischen 0,02 und 3 kg Wirkstoff oder mehr je ha. Die neuen Verbindungen können auch in Materialschutz u. a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung 4 werden in einer Mischung gelöst, die aus 25 Gewichtsteilen

Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung 5 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung 6 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung 8 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 9 werden mit 10 Teilen Natriumsalz eines Phenolsulfonsäureharnstoff-formaldehyd-Kondensats, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung 4 werden mit 2 Teilen Calciumsalz der Dodecylbenzolsulfonsäuren, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden. Beim Vermischem mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
- Ferridimethyldithiocarbamat,
- Zinkdimethyldithiocarbamat,
- Manganethylenbisdithiocarbamat,
- Mangan-Zink-ethylendiamin-bis-dithiocarbamat und
- Zinkethylenbisdithiocarbamat,
- Tetramethylthiuramdisulfide,
- Ammoniak-Komplex von Zink-(N,N-ethylen-bis-dithiocarbamat) und
- N,N'-Polyethylen-bis-(thiocarbamoyl)-disulfid,
- Zink-(N,N'-propylen-bis-dithiocarbamat),
- Ammoniak-Komplex von Zink-(N,N'-propylen-bis-dithiocarbamat) und
- N,N'-Polypropylen-bis-(thiocarbamoyl)-disulfid;

Nitroderivate, wie
- Dinitro-(1-methylheptyl)-phenylcrotonat,
- 2-sec-Butyl-4,6-dinitrophenyl-3,3-dimethylacrylat,
- 2-sec-Butyl-4,6-dinitrophenyl-isopropylcarbonat;

heterocyclische Substanzen, wie
- N-(1,1,2,2-Tetrachlorethylthio)-tetrahydrophthalimid,
- N-Trichlormethylthio-tetrahydrophthalimid,
- N-Trichlormethylthio-phthalimid,
- 2-Heptadecyl-2-imidazolin-acetat,
- 2,4-Dichlor-6-(o-chloranilino)-s-triazin,
- O,O-Diethyl-phthalimidophosphonothioat,
- 5-Amino-1-(bis-(dimethylamino)-phosphinyl)-3-phenyl-1,2,4-triazol,
- 5-Ethoxy-3-trichlormethyl 1,2,4-thiadiazol,
- 2,3-Dicyano-1,4-dithioanthrachinon,
- 2-Thio-1,3-dithio-(4,5-b)-chinoxalin,
- 1 (Butylcarbamoyl)-2-benzimidazol-carbaminsäuremethylester,
- 2-Methoxycarbonylamino-benzimidazol,
- 2-Rhodanmethylthio-benzthiazol,

4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und weitere Substanzen, wie
Dodecylguanidinacetat,
3-(3-(3,5-dimethyl-2-oxycyclohexyl)-2-hydroxyethyl)-glutaramid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Iod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze.

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-1-(4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Die folgenden Versuche 1, 2 und 3 zeigen die fungizide Wirkung der neuen Substanzen. Als Vergleichssubstanz diente die Verbindung (1-(2'-(2'',4''-Dichlorphenyl)-2'-(2''-propenyloxy)-ethyl)-1H-imidazol) (A).

## Versuch 1

### Wirksamkeit gegen Weizenmehltau

Blätter von in Töpfen gewachsenen Weizenkeimlingen der Sorte »Jubilar« wurden mit wäßrigen Emulsionen aus 80% (Gew.-%) Wirkstoff und 20% Emulgiermittel besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80% relativer Luftfeuchtigkeit aufgestellt. Nach 7 Tagen wurde das Ausmaß der Mehltauentwicklung ermittelt.

Das Ergebnis des Versuchs zeigte, daß beispielsweise die Wirkstoffe 1, 2, 3, 4, 5, 6, 8 und 9 bei Anwendung als 0,025%ige, 0,006%ige und 0,0015%ige Spritzbrühe eine bessere fungizide Wirkung hatten (beispielsweise 100%) als der Wirkstoff A (beispielsweise 60%).

## Versuch 2

### Wirksamkeit gegen Weizenbraunrost

Blätter von in Töpfen gewachsenen Weizensämlingen der Sorte »Jubilar« wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95%) gestellt. Während dieser Zeit keimten die Sporen aus und die Keimschläuche drangen in das Blattgewebe ein. Die infizierten Pflanzen wurden anschließend mit wäßrigen Spritzbrühen, die 80% Wirkstoff und 20% Emulgiermittel in der Trockensubstanz enthielten, tropfnaß gespritzt. Nach dem Antrocknen des Spritzbelages wurden die Versuchspflanzen

im Gewächshaus bei Temperaturen zwischen 20 und 22°C und 65 bis 70% relativer Luftfeuchte aufgestellt. Nach 8 Tagen wurde das Ausmaß der Rostpilzentwicklung auf den Blättern ermittelt.

Das Ergebnis des Vesuchs zeigte, daß beispielsweise die Wirkstoffe 1, 3, 4, 5, 6 und 9 bei Anwendung als 0,025%ige Spritzbrühe eine bessere fungizide Wirkung (beispielsweise 100%) hatten als der Wirkstoff A (beispielsweise 50%).

Versuch 3

Wirksamkeit gegen Gurkenmehltau

Blätter von in Töpfen gewachsenen Gurkenkeimlingen der Sorte »Chinesische Schlange« wurden im Zweiblattstadium mit einer Sporensuspension des Gurkenmehltaus (Erysiphe cichoracearum) besprüht. Nach etwa 20 Stunden wurden die Versuchspflanzen mit wäßriger Spritzbrühe, die 80% Wirkstoff und 20% Emulgiermittel enthielt, bis zur Tropfnässe besprüht. Nach dem Antrocknen des Spritzbelages wurden sie anschließend im Gewächshaus bei Temperaturen zwischen 20 und 22°C und 70 bis 80% relativer Luftfeuchtigkeit aufgestellt. Zur Beurteilung der Wirksamkeit der neuen Stoffe wurde das Ausmaß der Pilzentwicklung nach 21 Tagen beurteilt.

Das Ergebnis des Versuchs zeigte, daß beispielsweise die Wirkstoffe 1, 2, 4, 5, 6, 8 und 9 bei Anwendung als 0,025%ige Spritzbrühe eine gute fungizide Wirkung hatten (beispielsweise 95%).

**Patentansprüche für die Vertragsstaaten: BE, CH, DE, FR, GB, IT, LI, NL, SE**

1. Verbindung der allgemeinen Formel 1

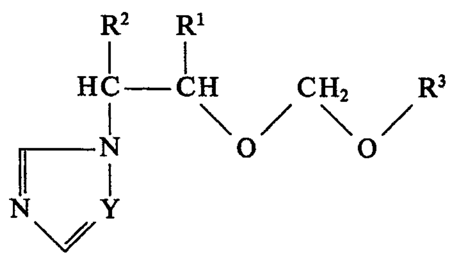

(1)

in der

$R^1$ tertiär Butyl oder Phenyl,
$R^2$ Wasserstoff oder Phenyl, Phenoxyl oder Thiophenoxyl bedeutet, wobei der Phenyl-, Phenoxyl- oder Thiophenoxylrest gegebenenfalls einfach oder mehrfach durch Halogen oder Phenoxy substituiert ist,
$R^3$ Methyl und
Y N bedeutet.

2. Fungizid, enthaltend eine Verbindung der Formel 1 gemäß Anspruch 1.

3. Fungizid, enthaltend einen festen oder flüssigen Trägerstoff und eine Verbindung der Formel 1 gemäß Anspruch 1.

4. Verfahren zur Herstellung eines Fungizids, dadurch gekennzeichnet, daß man einen festen oder flüssigen Trägerstoff vermischt mit einer Verbindung gemäß Anspruch 1.

5. Verfahren zur Bekämpfung von Pilzen, dadurch gekennzeichnet, daß man die Pilze oder die vor Pilzbefall zu schützenden Gegenstände mit einer Verbindung gemäß Anspruch 1 behandelt.

6. Verfahren zur Herstellung einer Verbindung gemäß Anspruch 1, dadurch gekennzeichnet, daß man ein Alkoholat der allgemeinen Formel 2

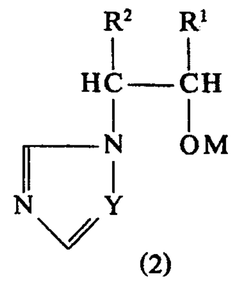

+ $Hal-CH_2-O-R^3 \longrightarrow$ (1)

(2) (3)

mit einem halogenierten Ether der Formel 3

$Hal-CH_2-O-R^3$ (3)

umsetzt, wobei $R^1$, $R^2$, $R^3$ und Y die im Anspruch 1 angegebene Bedeutung haben, Hal ein Halogenatom und M ein Alkali-, ein quartäres Ammonium- oder ein Phosphoniumion ist.

**Patentansprüche für den Vertragsstaat: AT**

1. Fungizid, enthaltend eine Verbindung

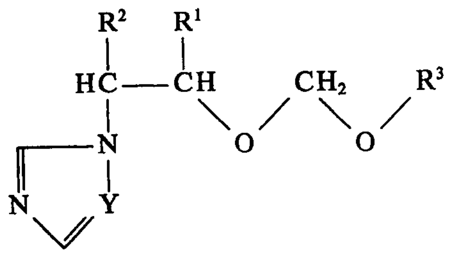

(1)

in der

$R^1$ tertiär Butyl oder Phenyl,
$R^2$ Wasserstoff oder Phenyl, Phenoxyl oder Thiophenoxyl bedeutet, wobei der Phenyl-, Phenoxyl- oder Thiophenoxylrest gegebenenfalls einfach oder mehrfach durch Halogen, Phenyl oder Phenoxy substituiert ist,
$R^3$ Methyl und
Y N bedeutet.

2. Fungizid, enthaltend einen festen oder flüssigen Trägerstoff und eine Verbindung gemäß Anspruch 1.
3. Verfahren zur Herstellung eines Fungizids, dadurch gekennzeichnet, daß man einen festen oder flüssigen Trägerstoff vermischt mit einer Verbindung gemäß Anspruch 1.
4. Verfahren zur Bekämpfung von Pilzen, dadurch gekennzeichnet, daß man die Pilze oder die vor Pilzbefall zu schützenden Gegenstände mit einer Verbindung gemäß Anspruch 1 behandelt.
5. Verfahren zur Herstellung einer Verbindung gemäß Anspruch 1, dadurch gekennzeichnet, daß man ein Alkoholat der allgemeinen Formel 2

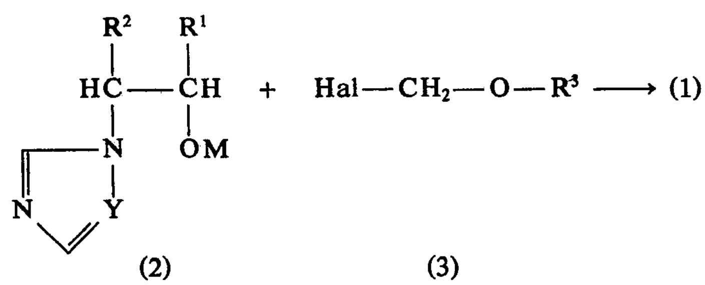

(2) (3)

mit einem halogenierten Ether der Formel 3

$Hal-CH_2-O-R^3$ (3)

umsetzt, wobei $R^1$, $R^2$, $R^3$ und Y die im Anspruch 1 angegebene Bedeutung haben, Hal ein Halogenatom und M ein Alkali-, ein quartäres Ammonium- oder ein Phosphoniumion ist.

**Claims for the Contracting states: BE, CH, LI, DE, FR, GB, IT, NL, SE**

1. A compound of the general formula 1

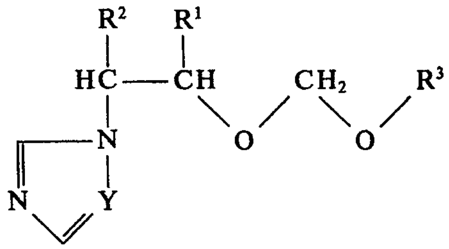

(1)

where

$R^1$ is tert.-butyl or phenyl,
$R^2$ is hydrogen, phenyl, phenoxy or thiophenoxy, phenyl, phenoxy or thiophenoxy being optionally mono- or polysubstituted by halogen or phenoxy,
$R^3$ is methyl, and
Y denotes N.

2. A fungicide containing a compound of the formula 1 as claimed in claim 1.
3. A fungicide containing a solid or liquid carrier and a compound of the formula 1 as claimed in claim 1.
4. A process for producing a fungicide, wherein a solid or liquid carrier is mixed with a compound as claimed in claim 1.
5. A process for combating fungi, wherein the fungi or the objects to be protected against fungal attack are treated with a compound as claimed in claim 1.
6. A process for the preparation of a compound as claimed in claim 1, wherein an alcoholate of the general formula 2

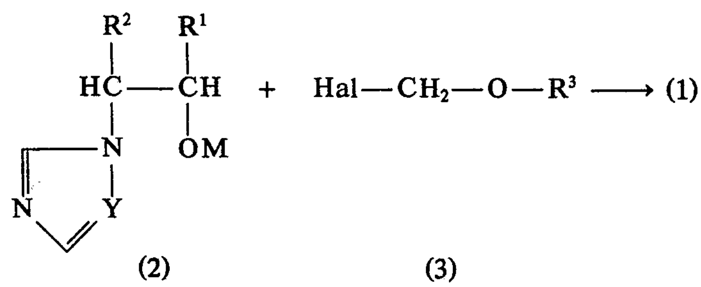

(2) (3)

is reacted with a halogenated ether of the formula 3

$$Hal-CH_2-O-R^3 \quad (3)$$

in which formulae $R^1$, $R^2$, $R^3$ and Y have the meanings given in claim 1, Hal is halogen, and M is an alkali metal ion, a quaternary ammonium ion or a phosphonium ion.

**Claims for the Contracting state: AT**

1. A fungicide containing a compound of the general formula 1

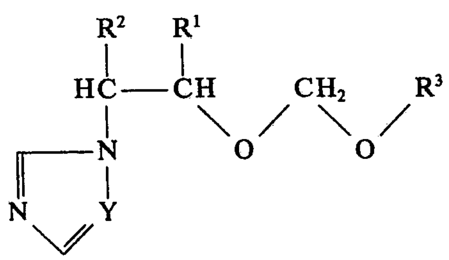

(1)

where

$R^1$ is tert.-butyl or phenyl,
$R^2$ is hydrogen, phenyl, phenoxy or thiophenoxy, phenyl, phenoxy or thiophenoxy being optionally mono- or polysubstituted by halogen or phenoxy,
$R^3$ is methyl, and
Y denotes N.

2. A fungicide containing a solid or liquid carrier and a compound as defined in claim 1.
3. A process for producing a fungicide, wherein a solid or liquid carrier is mixed with a compound as defined in claim 1.
4. A process for combating fungi, wherein the fungi or the objects to be protected against fungal attack are treated with a compound as defined in claim 1.
5. A process for the preparation of a compound as defined in claim 1, wherein an alcoholate of the general formula 2

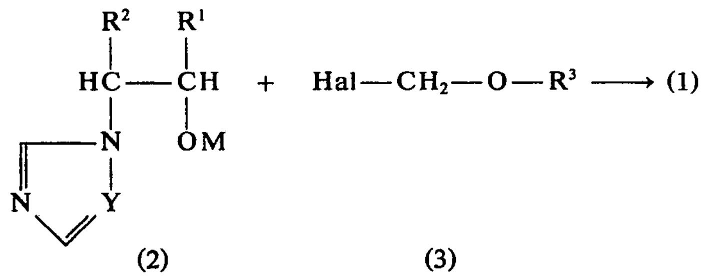

(2) (3)

is reacted with a halogenated ether of the formula 3

$$Hal-CH_2-O-R^3 \qquad (3)$$

in which formulae $R^1$, $R^2$, $R^3$ and Y have the meanings given in claim 1, Hal is halogen, and M is an alkali metal ion, a quaternary ammonium ion or a phosphonium ion.

**Revendications pour les Etats contractants: BE, CH, LI, DE, FR, GB, IT, NL, SE**

1. Composé de la formule générale 1

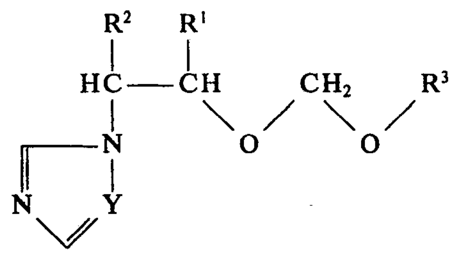

(1)

dans laquelle

$R^1$ désigne un groupe butyle tertiaire ou phényle,
$R^2$ désigne un atome d'hydrogène ou un groupe phényle, phénoxyle ou thiophénoxyle, le groupe phényle, phénoxyle ou thiophénoxyle pouvant éventuellement être substitué par un ou plusieurs atomes d'halogène ou groupes phénoxy,
$R^3$ désigne un radical méthyle et
Y = N.

2. Fongicide, contenant un composé de la formule 1 suivant la revendication 1.
3. Fongicide, contenant un composé de la formule 1 suivant la revendication 1 et une matière support solide ou liquide.
4. Procédé de préparation d'un fongicide, caractérisé en ce que l'on mélange un composé suivant la revendication 1 avec une matière support solide ou liquide.
5. Procédé de lutte contre les champignons, caractérisé en ce que l'on traite les champignons ou les objets à protéger des champignons avec un composé suivant la revendication 1.

6. Procédé de préparation d'un composé suivant la revendication 1, caractérisé en ce que l'on fait réagir un alcoolate de la formule générale 2 avec un éther halogéné de la formule 3

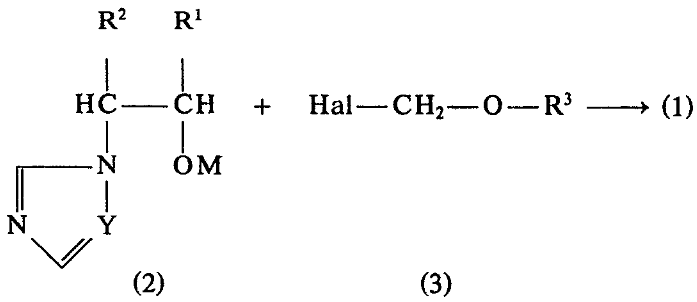

(2) (3)

dans lesquelles $R^1$, $R^2$, $R^3$ et Y possèdent la signification définie dans la revendication 1, Hal désigne un atome d'halogène et M un ion de métal alcalin, un ion ammonium quaternaire ou un ion phosphonium.

**Revendication pour l'Etat contractant: AT**

1. Fongicide, contenant un composé

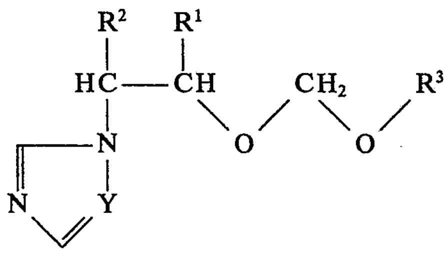

(1)

dans laquelle

$R^1$ désigne un groupe butyle tertiaire ou phényle,
$R^2$ désigne un atome d'hydrogène ou un groupe phényle, phénoxyle ou thiophénoxyle, le groupe phényle, phénoxyle ou thiophénoxyle pouvant éventuellement être substitué par un ou plusieurs atomes d'halogène ou groupes phénoxy,
$R^3$ désigne un radical méthyle et
Y = N.

2. Fongicide, contenant un composé suivant la revendication 1 et une matière support solide ou liquide.
3. Procédé de préparation d'un fongicide, caractérisé en ce que l'on mélange un composé suivant la revendication 1 avec une matière support solide ou liquide.
4. Procédé de lutte contre les champignons, caractérisé en ce que l'on traite les champignons ou les objets à protéger des champignons avec un composé suivant la revendication 1.
5. Procédé de préparation d'un composé suivant la revendication 1, caractérisé en ce que l'on fait réagir un alcoolate de la formule générale 2 avec un éther halogéné de la formule 3

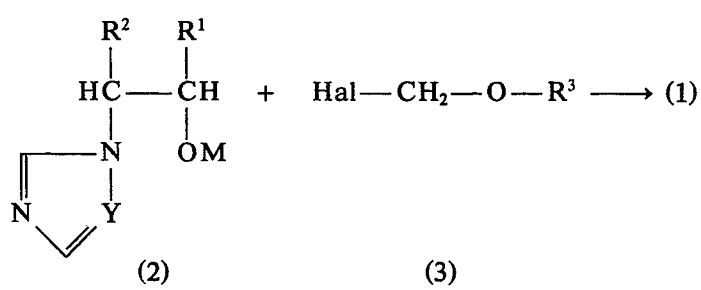

(2) (3)

dans lesquelles $R^1$, $R^2$, $R^3$ et Y possèdent la signification définie dans la revendication 1, Hal désigne un atome d'halogène et M un ion de métal alcalin, un ion ammonium quaternaire ou un ion phosphonium.